Europäisches Patentamt

European Patent Office

Office européen des brevets

(11) Publication number: 0 366 638
A2

# EUROPEAN PATENT APPLICATION

(21) Application number: 89850363.6

(22) Date of filing: 24.10.89

(51) Int. Cl.⁵: C07K 5/00 , A61K 37/02

(30) Priority: 27.10.88 SE 8803847

(43) Date of publication of application:
02.05.90 Bulletin 90/18

(84) Designated Contracting States:
AT BE CH DE ES FR GB IT LI NL SE

(71) Applicant: KabiGen AB

S-112 87 Stockholm(SE)

(72) Inventor: Sara, Vicki Rubian
Rörstrandsgatan 32
S - 113 40 Stockholm(SE)

(74) Representative: Henningsson, Gunnar et al
Bergling & Sundbergh AB Box 7645
S-103 94 Stockholm(SE)

(54) Neuromodulatory peptide.

(57) Peptide having the formula:
gly pro glu; gly pro; pro glu; ala gly pro; ala gly; gly pro glu thr; pro glu thr; glu thr, optionally blocked at one or both of the N- and C-terminals thereof;
the medicinal or diagnostic use of such peptide; and a composition for medicinal or diagnostic use comprising an active amount of such peptide.

*Fig. 1*

% Acetylcholine release

EP 0 366 638 A2

## Neuromodulatory peptide.

The present invention relates to new peptides having neuromodulatory capacity, and the invention also covers their medicinal or diagnostic use.

The present invention is based on the surprising discovery that certain peptides exhibit neuromodulatory activity by either stimulating or inhibiting neural activity within the central nervous system in mammals.

Accordingly, the present invention provides new peptides having the formula:
gly pro glu; gly pro; pro glu; ala gly pro; ala gly; gly pro glu thr; pro glu thr; or glu thr.

The peptide may, optionally, be blocked at one or both terminals thereof. Such blocking can be made with any suitable group, but it is preferred to block the H-terminal of the peptide by acylation, such as acetylation, and the Cterminal of the peptide by amidation, such as amidation using ammonia. Furthermore, any aminoacid not interfering with the neuromodulatory activity of the peptide can be used for blocking either the N-terminal or the C-terminal of the peptide.

Among preferred peptides there may be mentioned:
gly pro glu; gly pro; or pro glu, in particular the peptide gly pro glu is preferred for the purpose of the present invention.

As indicated above the peptide is medicinally or diagnostically useful. The preferred use is as a neuromodulator, for example in the treatment of disorders of the nervous system, in the treatment of senile dementia or in the treatment of psychiatric disorders.

The experimental work that has been done provide evidence that the peptide of the present invention is a modulator of neural function, thereby stimulating or inhibiting neural activity. Since the peptide functions as a neuromodulator its therapeutic use will apply to all situations where it is desirable to modulate neural activity. In the following there are given some examples of therapeutic applications using the peptide of the invention in order to modulate neural activity:
treatment of disorders of metabolism in the nervous system, e.g. catabolism and subsequent degeneration which occurs in dementia disorders, such as senile dementia of the Alzheimer type; anoxic and ischaemic brain damage, e.g. stroke and asphyxia;
treatment of neurological disorders, e.g. epilepsy;
treatment of disorders of neurotransmitter synthesis and function, e.g. learning and memory disorders, as well as psychiatric disorders, such as mania-depression and schizophrenia.

These are non-limiting examples of the therapeutic application of the peptide of the invention in the modulation of neural activity. In addition, the present peptide can be used diagnostically. Thus, it can be used to measure the presence of gly pro glu in a living organism, for example by immuno assay, so that neural activity can be quantitatively determined.

The therapeutic and diagnostic application of the peptide of the invention as a neuromodulatory compound will be related to the central nervous system, the peripheral nervous system, as well as the autonomic nervous system. Thus, it can be directed to all neural tissue or tissue of neural origin, including all cells which constitute the nervous system.

For medicinal or diagnostic use the peptide of the invention can be contained in a composition comprising an active amount of the peptide together with a therapeutically or diagnostically acceptable carrier therefor. For medicinal use pharmaceutical formulations are prepared from a predetermined quantity of the peptide of the invention, and such formulations may take the form of powders, elixirs, solutions, pills, capsules, pellets, tablets, sprays, snuffs, with anyone of a large variety of pharmaceutically acceptable vehicles or carriers. When in admixture with a pharmacutical vehicle or carrier the active ingredient, i.e. the peptide of the invention, usually comprises from about 0.001 to about 75%, normally from about 0.05 to about 15% by weight of the composition. Carriers, such as starch, sugar, talc, commonly used synthetic and natural gums, water and the like may be used in such formulations. The route of administration is not particularly critical and any suitable route can be used. Thus, subcutaneous, intramuscular or intravenous administration is conceivable as is administration directly into the central spinal fluid. Other preferred routes of administration are oral or nasal, in particular nasal administration, using drops, spray, snuff or the like. The composition may be an isotonic salt solution of the peptide of the invention, although other solvents or liquids may be used as a vehicle or carrier.

Relatively small quantities of the peptide of the invention, even as low as fractions of milligrams, may be used in administration to a patient. The active amount of the peptide of the invention should be of such order by weight that nanograms thereof can reach the target neural tissue.

The invention will now be described by non-limiting examples with reference to the appended drawings, wherein:
Fig. 1 is a diagram on the release of acetylcholine from cortical brain slices;

Fig. 2 illustrates stimulation and inhibition of cortical neurone activity upon administration of the peptide of the invention; and

Fig. 3 is a diagram illustrating the electrophysiological reflex response in the spinal cord upon administration of the peptide.

## EXAMPLE 1

The tripeptide glycylprolylglutamic acid ($H_2N$-Gly-Pro-Glu-COOH) was synthesized according to the stepwise solid phase technique (Kent, S.B.H. (1988) Ann.Rev.Biochem. 57, 957-989) in an Applied Biosystems Model 430 A peptide synthesizer. A phenylacetamidomethyl (PAM) resin was used as the solid support and the following tert-butyloxycarbonyl (tBoc) amino acid derivatives were employed: L-Glu-benzyl ester (OBzl), L-Pro and Gly. A standard program including pre-formation of symmetric anhydrides was used for the synthesis. The resulting peptide was cleaved from the resin and deprotected by the hydrogen fluoride (HF) method and subsequently purified by reverse phase high performance liquid chromatography (HPLC). The identity and purity of the final product was assessed by amino acid analysis.

## EXAMPLE 2

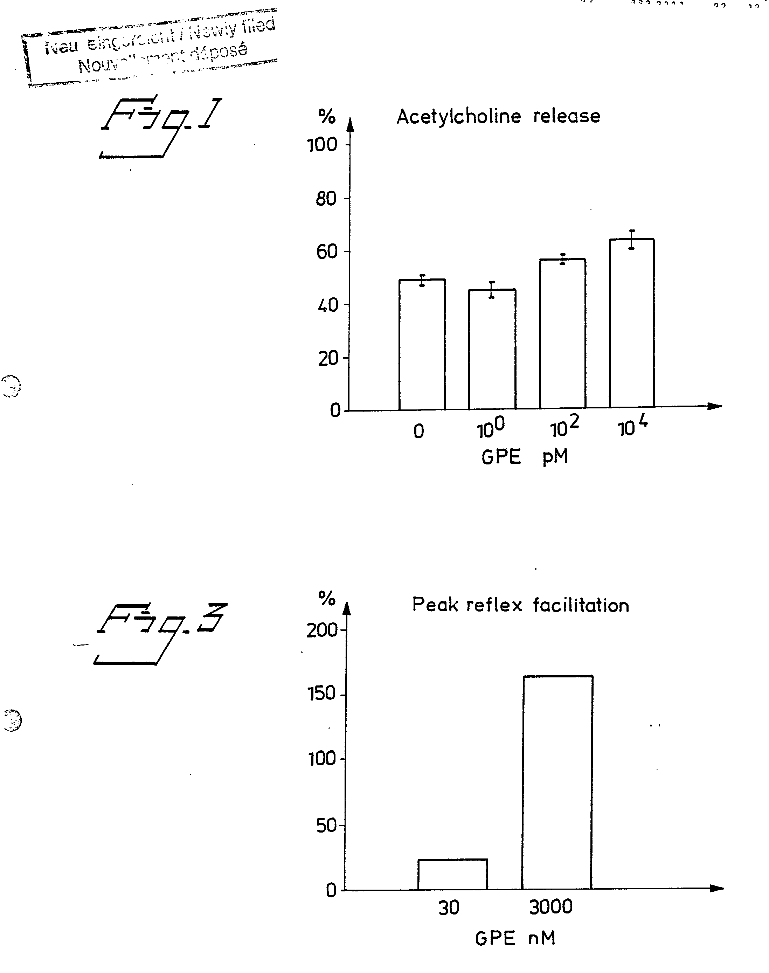

The tripeptide resulting from the manufacture according to Example 1 above was used in a test system according to L. Nilsson, V.R. Sara and A. Nordberg, Neurosience Letters, 1988, Vol. 88, pp 221-226. This test procedure is based on stimulation of the release of acetylcholine from cortical brain slices. The results of this experiment is shown in Fig. 1, wherein the tripeptide's ability to inhance the release of acetylcholine from neurones following depolarization is shown.

## EXAMPLE 3

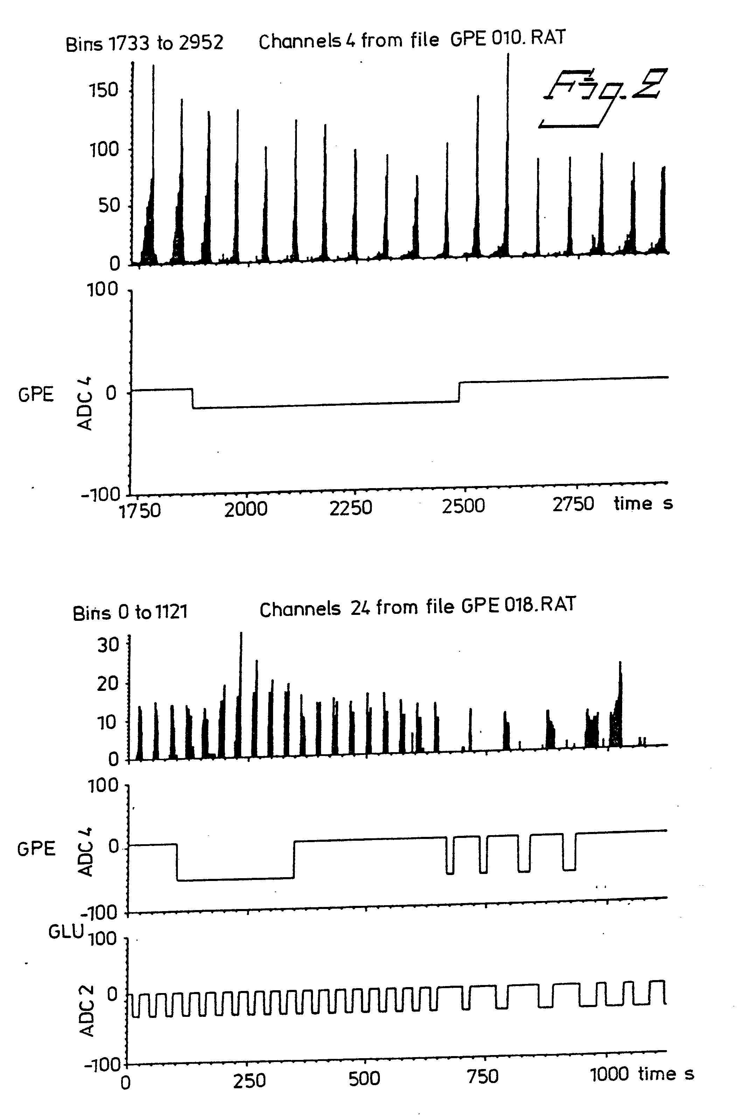

The tripeptide from Example 1 above was used in modulation of the electrophysiological activity of cortical neurones, and the results of this experiment is illustrated in Fig. 2. As can be seen from Fig. 2 the tripeptide both stimulates and inhibits cortical neurone activity and modulates the neural response to neurotransmitters. In this experiment the procedure according to T.W. Stone (Ed.), "Microiontophonesis and pressure ejection", John Wiley & Sons, New York, 1985 was used.

## EXAMPLE 4

Following the procedure of Z. Wiesenfeld-Hallin, Bruin Research 372:172-175, 1986, experiments were performed, wherein the tripeptide of Example 1 was shown to facilitate the electrophysiological reflex response in the spinal cord.

The result of these experiments is shown in Fig. 3, from which it is clear that the tripeptide increases both the peak and the duration of the reflex response.

## Claims

1. Peptide for medicinal or diagnostic use, characterized by the formula:
gly pro glu; gly pro; pro glu; ala gly pro; ala gly; gly pro glu thr; pro glu thr; glu thr, optionally blocked at one or both of the N- and C-terminals thereof.

2. Peptide according to claim 1, characterzied by the formula:
gly pro glu; gly pro; or pro glu.

3. Peptide according to claim 2 having the formula:
gly pro glu.

4. Peptide according to any preceding claim for use as a neuromodulator.

5. Peptide according to claim 4 for use in the treatment of disorders of the nervous system.

6. Peptide according to claim 4 for use in the treatment of senile dementia.

7. Peptide according to any preceding claim for use in the treatment of psyciatric disorders.

8. A composition for medicinal use comprising an active amount of a peptide according to any preceding claim in combination with a therapeutically acceptable carrier therefor.

9. A composition for diagnostic use comprising an active amount of a peptide according to any of claims 1 to 3 in combination with a diagnostically acceptable carrier therefor.

10. A composition according to claim 8, wherein said carrier is adapted for nasal administration.

Fig. 1

Acetylcholine release

Fig. 3

Peak reflex facilitation

Bins 1733 to 2952    Channels 4 from file GPE 010. RAT

*Fig. 2*

GPE    ADC 4

1750    2000    2250    2500    2750    time s

Bins 0 to 1121    Channels 24 from file GPE 018. RAT

GPE    ADC 4

GLU    ADC 2

0    250    500    750    1000 time s